**Europäisches Patentamt**

**European Patent Office**

**Office européen des brevets**

(19)

(11) Publication number : **0 407 789 B1**

(12) # EUROPEAN PATENT SPECIFICATION

(45) Date of publication of patent specification :
**22.09.93 Bulletin 93/38**

(51) Int. Cl.⁵ : **C12Q 1/68, // C07H21/04**

(21) Application number : **90111912.3**

(22) Date of filing : **22.06.90**

(54) **Nucleic acid detection method.**

(30) Priority : **23.06.89 JP 159717/89**
**13.06.90 JP 152610/90**

(43) Date of publication of application :
**16.01.91 Bulletin 91/03**

(45) Publication of the grant of the patent :
**22.09.93 Bulletin 93/38**

(84) Designated Contracting States :
**AT BE CH DE DK ES FR GB GR IT LI LU NL SE**

(56) References cited :
**EP-A- 0 235 726**
**EP-A- 0 237 833**

(73) Proprietor : **CANON KABUSHIKI KAISHA**
**30-2, 3-chome, Shimomaruko, Ohta-ku**
**Tokyo (JP)**

(72) Inventor : **Kato, Kinya, c/o Canon Kabushiki Kaisha**
**30-2, 3-chome Shimomaruko**
**Ohta-ku, Tokyo (JP)**
Inventor : **Iwashita, Harumi, c/o Canon Kabushiki Kaisha**
**30-2, 3-chome Shimomaruko**
**Ohta-ku, Tokyo (JP)**
Inventor : **Yamamoto, Nobuko, c/o Canon Kabushiki Kaisha**
**30-2, 3-chome Shimomaruko**
**Ohta-ku, Tokyo (JP)**
Inventor : **Sakuranaga, Masanori, c/o Canon Kabushiki Kaisha**
**30-2, 3-chome Shimomaruko**
**Ohta-ku, Tokyo (JP)**

(74) Representative : **Bühling, Gerhard, Dipl.-Chem. et al**
**Patentanwaltsbüro, Tiedtke-Bühling-Kinne & Partner, Bavariaring 4**
**D-80336 München (DE)**

EP 0 407 789 B1

**Description**

BACKGROUND OF THE INVENTION

Field of the invention

This invention relates to a nucleic acid detection method utilizing the hybrid formation reaction with two kinds of nucleic acids having complementarity, and a method for detecting a gene inherent in gene disease utilizing said method.

Related Background Art

When single stranded DNA's or RNA's have complementarity to each other, the portions having complementarity are bonded to become a double-strand, thereby forming a hybrid. As the methods for detection or quantification of nucleic acids utilizing the hybridization reaction, there may be included various methods such as the Southern method, etc., which have become one basic technique in various genetic engineering methods such as cloning of gene, gene recombination, screening of desired gene or diagnosis of disease by use of test sample gene.

As the analytical method utilizing hybridization of nucleic acid, there has been generally employed the method, in which a probe comprising DNA or RNA is applied with a label for detection of a hybrid, the labelled probe is hybridized with a sample nucleic acid, and the hybrid formed is detected by utilizing the label imparted to the probe.

As the method for labelling probe, there have been employed the method in which a radioisotope is introduced into probe, or the method in which a compound necessary for light-emission reaction, color formation reaction, fluorescent reaction, etc. is introduced into or bound to probe.

As the probe, for example, nucleic acid fragments directly separated from animals, plants, microorganisms, etc., cloned nucleic acid fragments cloned according to predetermined standards, oligonucleotides synthesized by a synthesizer, etc. have been utilized depending on the purpose, etc.

With development of genetic engineering, utilization frequency and application range of the analytical method of nucleic acids utilizing hybridization are also enlarging, and it has been increasingly demanded to have a method which can perform analysis with good sensitivity by simpler operations.

For example, in case of labelling by use of a radioisotope, it has the advantage of having good detection sensitivity of hybrid, etc., while expensive reagents, special instruments, devices, etc. therefor are required, because of handling of a radioactive substance, and also the operations are accompanied with danger.

On the contrary, non-radioactive labelling as represented by labelling with an enzyme utilizing the fluorescent reaction of biotin-avidin-anti-avidin antibody-fluorescent dye complex, while it has the advantage of performing labelling and detection by safe and simple operations, has the drawback that detection sensitivity is liable to be lower when employed for labelling of probe.

Also, in various analyses, nucleic acid fragment having a nucleotide chain length of 100 bases or more may be frequently employed as the probe to be labelled. Although such relative longer nucleic acid fragment has the advantage that it can be labelled with relative ease, synthesis by a synthesizer can be done with difficulty, and therefore there is the drawback that cumbersome workings such as separation operation from living body, cloning, etc. are required for its preparation.

On the contrary, a nucleic acid fragment with a length of less than 100 bases has the advantage that it can be synthesized easily by a synthesizer. However, it has the drawback that labelling, particularly labelling by a nick translation or an enzyme cannot be easily done.

Further, when it is desired to analyze plural kinds of nucleic acids by use of plural kinds of probes at the same time, for discrimination of which probe is contained in the hybrid formed, it is necessary to apply labels different from each other to the probes corresponding to the respective nucleic acids. However, such operations are extremely cumbersome.

On the other hand, application of the analytical method of nucleic acid utilizing hybridization for the detection method of a gene related to disease is now being investigated.

As the diseases induced by mutation, etc. at gene level, there have been known various gene diseases, for example, hereditary hemoglobin abnormal diseases such as phenylketonuria, thalassemia, etc., OTC (ornitine transcarbamylase) deficiency, Duchenne type muscular dystrophy, etc.

Also, there is a report that mutation of a protein by gene mutation participates in generation of a certain kind of cancer.

Or, the probability is increasing that serious diseases such as AIDS, ATL, etc. are caused to occur by retrovirus.

Diagnosis of a gene disease has been generally done by measuring the activity of the enzyme and the amount of the protein characteristic of the gene disease and judging where there is deficiency or abnormality in these from the results, but in some cases, the enzyme which becomes the index of the disease may exist locally at the site of organs, etc. from which sampling can be done with difficulty or a test sample may be available with difficulty, and also the index enzyme to be measured cannot be sometimes identified, and these diagnosis methods are not necessarily satisfactory.

On the other hand, diagnosis of viral infectious disease has been done exclusively by the ELISA method and the PA method, but these methods are not necessarily correct.

If a gene disease, cancer, viral infectious disease can be found at early stage by diagnosis at gene level for which sampling of a test sample is relatively easier and correct examination can be expected, adequate therapy can be done at early stage. Thus, it has been increasingly demanded to have a diagnosis method at gene level.

As one of such effective methods, there is, for example, RFLP (restriction fragment length polymorphism).

This method is a method in which the DNA fragment obtained by cleavage of the whole gene of human being with restriction enzymes is developed by agarose gel electrophoresis, the electrophoresed fragment is hybridized with a probe DNA labelled with radioisotope, etc., and the pattern of electrophorogram of the hybrid detected by utilizing the label is compared with that of electrophorogram obtained similarly for the normal gene, thereby detecting the gene related to the disease.

The diagnosis method by analysis of gene utilizing RFLP, etc. is given attention as an extremely useful method in practicing a surer diagnosis.

However, in practice of these methods, the testing personnel is required to have molecular biological knowledges and skilled technique, and yet the operations are also cumbersome, and therefore persons engaged in medicine such as physicians, examination engineers, etc. cannot easily practice the method.

Also, in these methods it takes about one day for each step of cleavage of DNA, Southern blotting, hybridization, and it takes a long time for obtaining the final result, whereby the number of test samples handled per one examination is limited and therefore it is a very difficult working to examine DNA's of many patients over multiple items.

Also, application of the detection method utilizing hybridization of nucleic acids to the taxonomic method of microorganisms such as bacteria, etc., particularly for assay of pathogens, attracts notice.

Taxonomic identification of microorganisms has been practiced by investigating the ecological properties and biochemical properties of microorganisms by comparison with the standard microorganisms.

However, according to such a method, there are some cases that judgement of the properties may differ depending on the examination method or that the identification results may differ depending on which property is important.

In the case when the test sample is bacteria obtained from a patient having caused an infectious disease, if there is an error in the identification results, no adequate corresponding treatment can be done, and hence a surer identification is particularly required.

Accordingly, in recent years, in order to provide a surer identification method, there have been made attempts to use the DNA-DNA hybridization method for detection and identification of causative bacteria in bacterial infectious diseases.

In this method, a characteristic base sequence of said bacteria in DNA's of bacteria is made the standard sequence, and whether a base sequence highly homologous with said standard sequence exists in the DNA extracted from the test sample bacteria or not is examined according to the hybridization method by use of a probe capable of detecting said standard sequence, thereby performing taxonomic identification of the test sample bacteria.

As the probe in this method, the DNA fragment cloned from the chromosome of bacteria, the plasmid itself possessed by the bacteria, synthetic oligonucleotides, etc. may be employed.

Specifically, there may be included the following methods, in which the probe employed is:

1) a cloned random fragment;

2) a ribosome RNA; and

3) a synthetic oligonucleotide having a base sequence characteristic of the species or the genus selected from the base sequence of ribosome RNA.

As the above method 1), a method, in which random fragments obtained by cleavage of DNA extracted from bacteria with an appropriate restriction enzyme are cloned, and the clone specifically reactive only with the desired taxonomic group from among them is selected and used as the probe, has been known from, for example, Criale, M.S., Flowers, R.S. et al.: DNA probes, 143-148, 1986, etc.

As the above method 2), there has been known a method disclosed by Edelstein, P.H.; J. Clin. Microbiol.,

23:481-484, 1986. This is a method which is being watched in the fact that the ribosome RNA's of bacteria are relatively similar within the same taxonomic group, and there is the advantage that the detection sensitivity becomes higher by use of ribosome RNA, because a large number of gene copies exists in the chromosome of bacteria.

As the above method 3), there have been reported examples in which base sequences specific to the genus, the species or the subspecies, etc. belonging to the genus Proteus selected from the base sequence of the ribosome RNA of the genus Proteus are utilized for probe (Haun, G. & Gobel, U.; FEMS Microbiol. Lett., 43: 187-194, 1987).

However, also these methods have commonly the problems in the various analytical methods for nucleic acids utilizing hybridization as previously described.

EP-A-0 235 726 discloses a method for the determination of particular nucleic acid sequences in a sample nucleic acid in which the sample itself is labelled. Probe nucleotide lengths of at least 700 base pairs are described.

EP-A-0 237 833 discloses solution phase hybridisation assays for detecting polynucleotide sequences, in which the sequence to be detected contains a reactive site and the probe is labelled, or vice versa. A solid phase, to which a reactive partner for the reactive site is bound, is then used to separate the hybrid from the remaining solution and the label is measured in either the immobilised fraction or in the solution.

## SUMMARY OF THE INVENTION

The present invention has been accomplished in view of the problems in various detection methods of the prior art utilizing the hybridization method as described above, and its object is to provide a nucleic acid detection method utilizing the hybridization method which can be practiced simply and correctly.

Here, the present invention provides a nucleic acid detection method to detect the presence of a nucleic acid to be detected in a sample nucleic acid comprising the steps of

(a) providing a probe nucleic acid which is an oligonucleotide with a nuclotide chain length of 25 bases or less capable of detecting a point mutation of a genetic disease, immobilized on a carrier;

(b) labelling the said sample nucleic acid;

(c) adding the labelled sample nucleic acid to the immobilized probe nucleic acid;

(d) detecting the point mutation by detecting the presence of the said nucleic to be detected in the said sample nucleic acid bound to the said probe nucleic acid.

## BRIEF DESCRIPTION OF THE DRAWINGS

Fig. 1 is a chart showing the arrangement of spots of the probe nucleic acid on a filter in Example 2; and

Fig. 2 is a chart showing the arrangement of spots of the probe nucleic acid on a filter in Example 3.

## DESCRIPTION OF THE PREFERRED EMBODIMENTS

The method of the present invention can be utilized for detection, quantification of various nucleic acids such as DNA or RNA derived from living bodies of human, animals, plants, microorganisms such as bacteria, etc., fungi, protozoa, etc., recombinant DNA, synthesized DNA, etc., and kinds of nucleic acids which are the subject to be detected (nucleic acid to be detected) are not limited.

In the method of the present invention, firstly a nucleotide chain length of sample nucleic acid to be analyzed is controlled if necessary, and this is labelled.

For these sample nucleic acids, those extracted from natural products may be used, and for labelling of the sample nucleic acids, there can be utilized the methods generally utilized for labelling of probes, such as a labelling method by means of radioisotopes (RI), a method in which non-radioactive labelling substances (non-IR) such as enzymes or compounds necessary for inducing fluorescence, light-emission or color formation such as biotin, dinitrophenylnucleotide derivatives, etc. are introduced into or bound to sample nucleic acids and so on.

For labelling of sample nucleic acids, the terminal end labelling method, the substitution synthesis method, the nick translation method, etc. can be utilized.

When the same extent of labelling amount is used, non-RI label is generally lower in sensitivity as compared with RI label, but in the method of the present invention, detection with high sensitivity becomes possible even when a non-RI label may be employed.

In the method of the prior art, probe nucleic acids having a short nucleotide chain length are labelled and used in most cases, but in such cases, the label incorporating portion is limited, and the incorporation amount of label is limited.

On the contrary, in the method of the present invention, when the nucleotide chain length of a sample nucleic acid is long, the sample nucleic acid has many label incorporating portions, and therefore even in the case of labelling with non-RI, by increasing the label incorporation amount, its low sensitivity can be compensated, whereby detection with high sensitivity is rendered possible.

When the sample nucleic acid is double-stranded, it is formed into a single strand at an appropriate stage before effecting hybridization, for example, after labelling.

On the other hand, as the probe nucleic acid, any nucleic acid having a chain length of 25 bases or less which can be artificially synthesized conveniently by a DNA synthesizer can be readily utilized.

By use of a probe nucleic acid with such short nucleotide chain length, there are such advantages that synthesis, availability or preparation of the probe nucleic acid itself become extremely easy, that the reaction time for hydridization can be shortened, that the step of labelling onto the probe nucleic acid can be omitted, that detection with high sensitivity becomes possible, etc.

Utilization of a probe nucleic acid with short nucleotide chain length in the present invention is also rendered possible, partly because no label is applied to the probe nucleic acid, whereby no requirement necessary for labelling is demanded for a probe nucleic acid.

For example, in labelling according to the nick translation method or a binding method of a labelled enzyme, the nucleic acid to be labelled is required to have a nucleotide chain length of some extent or more, but the probe nucleic acid utilized in the present invention is not demanded to have such requirement. Therefore, it is readily available (prepared), and also, as described above, it becomes possible to utilize one with short nucleotide chain length capable of realizing detection with high sensitivity as the probe nucleic acid.

For the reaction between the probe nucleic acid and the sample nucleic acid, the method of contacting an immobilized probe nucleic acid with a sample nucleic acid can be suitably utilized.

For immobilization of a probe nucleic acid, it is possible to utilize various methods which have been utilized for immobilization of nucleic acids, such as the method in which probe nucleic acid is immobilized onto an appropriate carrier for immobilization such as nitrocellulose, nylon film, etc. by utilizing the physical adsorption method or chemical reaction.

Hybridization of a probe nucleic acid with a labelled sample nucleic acid can be effected following conventional methods.

For example, the conditions of hybrid formation reaction depend on the nucleotide chain length and the base sequence possessed by the probe nucleic acid. Generally speaking, it is carried out in a hybridization solution containing formamide, an appropriate salt and Denhardt solution, while controlling the temperature.

Then, by washing the carrier with solutions of different conditions such as a solution, a salt concentration of which is lowered, or a solution, temperature of which is elevated, DNA which forms no hybrid can be washed away from the carrier.

As the conditions for the hybrid formation reaction and the conditions for washing, the optimum conditions corresponding to the desired object may be conveniently selected.

Detection of the hybrid formed may be practiced according to the operations corresponding to the kind of the label employed.

Also, by analyzing quantitatively the amount of label possessed by the hybrid formed, the nucleic acid to be detected can be quantified.

In the operations as described above, by use of plural kinds of nucleic acids as the probe nucleic acid, when a plural number of nucleic acids to be detected are contained in the sample nucleic acid, they can be detected at the same time.

More specifically, plural kinds of probe nucleic acids corresponding to the respective plural kinds of nucleic acids to be detected are immobilized at a predetermined arrangement, so as to be seen which probe nucleic acid is positioned at which position, then the immobilized probe nucleic acids are reacted with a labelled sample nucleic acid. After completion of the reaction, the formation position of the hybrid is detected by utilizing the label, and the kind of the corresponding probe nucleic acid is identified from the position indicating positiveness, and from the result the kind of the nucleic acid to be detected contained in the sample nucleic acid can be judged.

In the method using plural kinds of probe nucleic acids, it can be easily judged from the immobilized position previously set of probe nucleic acids that the sample nucleic acid has been hybridized with which probe nucleic acid since probe nucleic acids are immobilized at the positions previously set.

Therefore, it is not necessary to use different labels for respective probe nucleic acids so that the respective plural kinds of probe nucleic acids can be chosen as in the method of the prior art in which labelled probes are allowed to react with a sample nucleic acid.

As the probe nucleic acids when using plural kinds of probe nucleic acids, various nucleic acids can be utilized. For effecting the hybrid formation reaction with plural kinds of probe nucleic acids efficiently at the same time by the same operation, it is preferable that the nucleotide chain lengths of the respective probe nucleic acids should be controlled to the lengths necessary for the individual hybrid formation reactions in the plural kinds of probe nucleic acids to proceed under the same conditions.

Specifically, for example, the dissociation temperature (Tm: temperature for a single strand formation) of the hybrids to be formed in the individual hybrid formation reactions may be preferably made regular.

More specifically, for calculation of Tm value, various formulas have been employed depending on the hybridization solution employed. For example, in 0.9 M NaCl, Tm of an oligonucleotide of 20-mer or less is represented by:

$$Tm = 2 \times (A + T) + 4 \times (G + C)$$

wherein A, T, G and C respectively show the numbers of adenine, thymine, guanine, cytosine (see Continued Biochemical Experimental Course 1, Gene Study Method II, p. 236).

Accordingly, following this formula, the base sequences of the respective probe nucleic acids may be substituted in this formula for selection of their lengths so that the Tm values in the respective probe nucleic acids may be regular.

The nucleic acid detection method of the present invention as described above is useful for detection of genes inherent in gene diseases, cancers, viral infectious diseases.

As an example of detection of genes inherent in gene diseases, etc. according to the method of the present invention, the method including the processes shown below can be mentioned:

a) the process for immobilizing a probe nucleic acid related to the gene which is an index of the disease which is the examination item;

b) the process for labelling the test sample chromosome DNA (sample nucleic acid);

c) the process for reacting the immobilized probe obtained in the process a) with the labelled DNA obtained in the process b);

d) the process for detecting the hybrid formed in the process c) by utilizing the label.

As the nucleic acid probe used in the above process a), one having a base sequence necessary for recognizing the base sequence inherent in a gene of gene disease, etc. may be employed, and its nucleotide length is not particularly limited.

For example, when a disease based on point mutation by substitution of one base pair is to be detected, in judging the difference of one base effectively and with good sensitivity, an oligonucleotide comprising 25 bases or less, more preferably 17 to 20 bases is employed as the nucleic acid probe.

Also, the substitution site corresponding to point mutation should be preferably arranged so as to be positioned at the center of the oligonucleotide as the probe nucleic acid for performing more accurate judgement.

In the process a), examinations over plural items can be done at the same time by immobilizing the plural kinds of probe nucleic acids corresponding to the plural examination items at a predetermined arrangement on the same carrier.

Specific operations can be done following the method by use of plural kinds of probes as previously described.

As the test sample DNA in the process b), a chromosome DNA itself, a chromosome DNA cleaved to a suitable length with an appropriate restriction enzyme, etc. can be utilized.

When multiple items of examination are to be performed at the same time, it is efficient to utilize fragments cleaved to suitable lengths with a restriction enzyme.

Also, for labelling, the methods as mentioned above, etc. can be utilized.

The hybrid formation reaction in the process c) can be performed according to the method as previously described.

When point mutation is to be detected, only one difference of nucleotide may sometimes fail to differentiate presence and absence of a point mutation due to generation of mismatch between the probe and the nucleic acid.

However, Tm of hybrid is influenced by the base pair number not complementary in the mismatched hybrid, the contents of G (guanine) and C (cytosine) in the probe nucleic acid, the nucleotide chain length of the probe nucleic acid, the position of point mutation, the kind of mismatch, etc., then it is important to select the temperature conditions, etc. which are free from generation of mismatch hybrid interfering with detection of the desired point mutation, corresponding to the constitution of the probe nucleic acid employed, etc.

For example, Tm drops by 5 to 10°C per one base pair which is not complementary contained in a mismatch hybrid.

Therefore, the temperature of hybridization is required to be selected in view of the Tm of the desired hybrid and the Tm of the mismatch hybrid which can be formed.

Also, when plural kinds of probe nucleic acids are used as immobilized on the same carrier, in addition to the requirement concerning the mismatch as described above, these nucleotide chain lengths, etc. should be preferably controlled so that the Tm of the hybrids formed with the respective probe nucleic acids may be regular.

The above-mentioned process d) can be performed according to the method corresponding to the label employed.

In the method as described above, by using a nucleic acid having a base sequence inherent in a microorganism such as bacteria, virus, etc. as the probe, and a nucleic acid separated from a microorganism as the sample, microorganisms can be identified taxonomically.

In the case of taxonomy of bacteria, the probe nucleic acid having a nucleotide chain length of about 20-mer, while in the case of taxonomy of virus, one having a nucleotide chain length of about 20-mer may be suitably employed.

Example 1

(N.B. The probes and methods referred to in Examples 1,2,4 and 5 do not from part of the invention as claimed)

1-1) Immobilization of probe nucleic acid:

A 41-mer oligonucleotide having the following DNA base sequence constituting a part of a plasmid pUC 19 was synthesized by a DNA synthesizer (a product of ABI, Model 381 A):

$$5'\text{GATCGCCCTTCCCAACAGTTGCGCAGCCTGAATGGCGAATG}3'.$$

By use of a part of the synthesized product, its purity was examined by 15 % polyacrylamide gel electrophoresis containing 7 M urea. As the result, the purity of the synthesized product was judged to be 90 % or higher, and therefore the synthesized product was used directly without purification in the subsequent operations.

A solution for spotting of the synthesized product at 200 ng/$\mu$l concentration was prepared, and each 2 $\mu$l of the solution was spotted onto a nitrocellulose filter (a product of Schleicher & Schanel), dried and then baked at 80 °C for 2 hours.

1-2) Labelling of sample nucleic acid and preparation of hybridization solution:

As sample nucleic acids, a plasmid pBR 322 (Sample A), a plasmid pUC 19 (Sample B) and a mixture of the plasmid pBR 322 and the plasmid pUC 19 (Sample C, mixing weight ratio 1:1) were prepared, and labelling of the sample and preparation of the hybridization solution were conducted according to the following operations by using individually these samples.

With 2 $\mu$l of a solution containing 1 $\mu$g of Sample A, B or C were mixed 2.0 $\mu$l of 10 x TA buffer and 16.0 $\mu$l of water, and to the mixture obtained was added 10 units of HindIII (a product of TOYOBO), and the mixture was left to stand at 37 °C for 2 hours to carry out the reaction.

Next, 2.0 units of T4DNA polymeraze (a product of TOYOBO) was added, followed further by the reaction at 22 °C.

After one hour, to the reaction mixture were added 2mM dATP 2.0 $\mu$l, 2 mM dCTP 2.0 $\mu$l and 2 mM dGTP 2.0 $\mu$l (products of TOYOBO), and further 6.0 $\mu$l of biotinylated UTP (a product of BRL), and the mixture was left to stand at 37 °C.

After 40 minutes, the reaction was stopped by addition of 10 $\mu$l of 100 mM EDTA into the reaction mixture, and the reaction mixture subjected to heating treatment at 95 °C for 5 minutes to convert the product into a single strand.

The reaction mixture subjected to the heating treatment was mixed with 100 % formamide 9 ml, 20xSSC 5 ml, 50 x Denhardt solution 0.4 ml, 1M sodium phosphate buffer (pH 6.5) 0.4 ml and sonication-modified salmon sperm DNA (a product of Sigmer) of 50.0 mg/ml concentration 0.1 ml to obtain a hybridization solution.

According to the operations as described above, three kinds of hybridization solutions prepared from Sample A, B and C respectively were obtained.

1-3) Hybridization:

By using individually the three kinds of hybridization solutions obtained in the operation of the above item 1-2), hybridization was effected according to the following operations.

First, a filter having the probe nucleic acid previously prepared immobilized in spots was dipped in a mixture of 200 ml of a mixture of 3 x SSC and Denhardt solution (mixing volume ratio 1:1) at 42 °C for 30 minutes to effect pre-hybridization, then the hybridization solution (3 ml) of Sample A, B or C was added therein, and the mixture was sealed within a polyethylene bag and left to stand at 42 °C.

After 12 hours, the filter was taken out, washed and the color indication reaction was carried out in conventional manner (Bioindustry, Vol. 3, No. 6, 1989, p479-504, etc.).

As the result, when the hybridization solution from Sample B was employed and when the hybridization solution from Sample C was employed, color formation of blue violet was recognized at the spot of probe nucleic acid on the filter, whereby presence of pUC 19 was confirmed in samples B and C.

Example 2

2-1) Immobilization of probe nucleic acid:

A 41-mer oligonucleotide having the following DNA base sequence constituting a part of a plasmid pUC 19 (Probe A) and a 41-mer oligonucleotide having the following DNA base sequence constituting a part of a plasmid pBR 322 (Probe B) were synthesized by a DNA synthesizer (a product of ABI, Model 381A).

## Probe A

$$5' \text{GATCGCCCTTCCCAACAGTTGCGCAGCCTGAATGGCGAATG}\, 3'$$

## Probe B

$$5' \text{CCGTTCAGCCCGACCGCTGCGCCTTATCCGGTAACTATCGT}\, 3'$$

When the purities of these synthesized products were examined according to the same operation item 1-1) in Example 1, both were found to have a purity of 95 % or more. These synthesized products were used directly without purification in the subsequent operations.

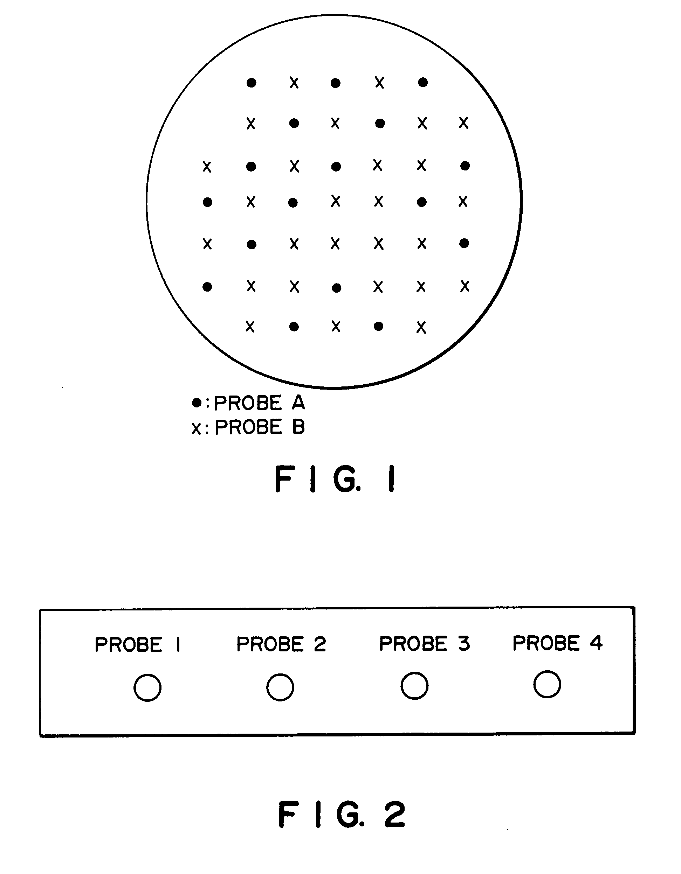

A solution for spotting of each of the synthesized products at 200 ng/μl concentration was prepared, and each 2 μl of the solution was spotted onto a nitrocellulose filter (a product of Schleicher & Schanel) so that the spots of Probe A and B were arranged as shown in Fig. 1, dried and then baked at 80 °C for 2 hours.

2-2) Labelling of sample nucleic acid and preparation of hybridization solution:

Sample nucleic acids were prepared as follows:

Sample D:

Mixture of plasmid pUC 19 and chromosome DNA prepared from E. coli JM109 strain (a product of TAKARA) in conventional manner (mixing weight ratio, 1:1).

Sample E:

Mixture of plasmid pBR 322 and chromosome DNA prepared from E. coli HB101 strain (a product of TOYOBO) in conventional manner (mixing weight ratio, 1:1).

Sample F:

Chromosome DNA prepared from E. coli HB101 strain in conventional manner.

By using these samples individually, labelling and preparation of hybridization solution of the respective Samples were conducted in the same manner as in the item 1-2) in Example 1 to obtain three kinds of hybridization solutions from Sample D, E and F.

2-3) Hybridization:

By using individually the three kinds of hybridization solutions obtained in the operation of the above item 2-2), pre-hybridization, hybridization and color formation reaction were carried out in the same manner as in the item 1-3) in Example 1.

As the result, color formation occurred at the spot portion of Probe A on the filter which had been reacted with the hybridization solution from Sample D, while color formation occurred at the spot portion of Probe B on the filter which had been reacted with the hybridization solution from Sample E. On the contrary, no color formation was recognized at the spot portion on the filter which had been reacted with the hybridization solution from Sample F.

Example 3

Among mutations of PAH gene seen in leukocyte DNA of phenylalaniuria patient, as mutations of high frequency (∗ portion), the following ones have been known.

**Haplotype 3**

Normal:     $5'$TCCATTAACAGTAAGTAATTT$^{3'}$ (Probe 1)

Abnormal:   $5'$TCCATTAACA**A**TAAGTAATTT$^{3'}$ (Probe 2)
                              ∗

**Haplotype 2**

Normal:     $5'$CACAATACCTCGGCCCTTCTC$^{3'}$ (Probe 3)

Abnormal:   $5'$CACAATACCT**T**GGCCCTTCTC$^{3'}$ (Probe 4)
                            ∗

Accordingly, for utilizing these four kinds of oligonucleotides as probe nucleic acids, they were synthesized by a DNA synthesizer (a product of ABI, Model 381 A).

When the purities of the synthesized products were examined according to the same operation as in the item 1-1) in Example 1, all were found to have a purity of 95 % or more.

By using directly these synthesized products, according to the same operations as in the item 2-2) in Example 2, spots of the respective probes were formed with the arrangements shown in Fig. 2 on a nitrocellulose filter.

Next, from the cultured amniotic fluid cells from test sample donors arbitrarily selected, a double-stranded DNA was extracted in conventional manner and digested with Pvu II. The digested product obtained was reacted with photobiotin (a product of Bresatec) in conventional manner to be applied with biotin label, and then the double-stranded DNA was converted into a single strand by the heating treatment at 100 °C for 10 minutes.

2 μg of the labelled sample was added into a solution containing 5 x SSPE [50 mM sodium phosphate buffer (pH 7.0), 0.9 M NaCl, 5 mM EDTA], 0.3 % SDS and sonication-modified E. coli DNA (a product of Sigmer) of 10 μg/ml concentration to obtain a hybridization solution.

By use of the hybridization solution together with the probe-immobilized filter previously prepared, pre-hybridization and hybridization were conducted according to the same operations as in the item 1-3) in Example 1 except that the hybridization conditions were made 50 °C, 16 hours.

After completion of the reaction, the filter was subjected to washing twice with 2 x SSPE containing 0.1 % SDS at room temperature for 30 minutes, further once with 5 x SSPE containing 0.1 % SDS at 55 °C for 10 minutes.

When the filter after washing was subjected to the color formation reaction of biotinylated nucleic acid in conventional manner, color formation occurred at the spots of Probe 1 and Probe 3, and the test sample donor was judged to be suspected of no phenylalaninuria.

When the same operations as described above were conducted by use of cultured amniotic fluid cells from another test sample donor arbitrarily selected, color formation occurred at the spot of Probe 4, and hence the test sample donor was judged to be suspected of phenylalaninuria.

Example 4

By use of the experimental system according to an example of the present invention of Example 1, comparison was made with a prior art example. The probe employed is shown below, which is the same as in Example 1:

$$5'\text{GATCGCCCTTCCCAACAGTTGCGCAGCCTGAATGGCGAATG}3'.$$

Also, as the samples, the same three kinds of Sample A, B, C as in Example 1 adjusted to predetermined concentrations were employed.

As the sensitivity investigation according to the example of the present invention, a nitrocellulose filter spotted with probes similarly as in Example 1 was prepared.

As the sample, DNA applied with the same pre-treatment and labelling as in Example 1 was prepared, and hybridization solutions were prepared to have amounts of 50 ng, 5 ng, 0.5 ng, 0.05 ng and 0.005 ng of the DNA.

By use of the hybridization solution together with the probe-immobilized filter previously prepared, pre-hybridization and hybridization by use of the hybridization solutions with the respective concentrations were conducted according to the same operations as in the item 1-3) in Example 1 except that the hybridization conditions were made 50 °C, 16 hours.

After completion of the reaction, the filter was subjected to washing twice with 2 X SSPE containing 0.1% SDS at room temperature for 30 minutes, further once with 5 X SSPE containing 0.1 % SDS at 55 °C for 10 minutes.

When the filter after washing was subjected to the color formation reaction of biotinylated nucleic acid in conventional manner, the following results were obtained.

|   | 50ng | 5ng | 0.5ng | 0.05ng | 0.005ng |
|---|------|-----|-------|--------|---------|
| A | x | x | x | x | x |
| B | o | o | o | o | x |
| C | o | o | o | x | x |

Note:  o ... color formation

x ... no color formation

That is, it was evidenced that color formation occurred to about 0.05 to 0.5 ng to enable detection.

On the other hand, as the prior art example, firstly the probe was labelled. The labelling method was performed by synthesizing a complementary double-stranded DNA corresponding to the probe, introducing biotinylated dUTP at 3'-end with the T4 DNA Polymerase, and dissociating the labelled product into a single strand, which was used as the probe.

The sample nucleic acids A, B and C were spotted onto a nitrocellulose filter at the amounts shown below in Table 1.

| Filter No. | 1 | 2 | 3 | 4 | 5 |
|---|---|---|---|---|---|
| Sample | | | | | |
| | ng | ng | ng | ng | ng |
| A | 50 | 5 | 0.5 | 0.05 | 0.005 |
| B | 50 | 5 | 0.5 | 0.05 | 0.005 |
| C | 50 | 5 | 0.5 | 0.05 | 0.005 |

The five kinds of filters were dried and then oven-dried at 80 °C for 2 hours.

The probe previously prepared was subjected to heating treatment at 95 °C for 5 minutes to be converted into a single strand.

The reaction mixture subjected to the heating treatment was mixed with 100 % formamide 9 ml, 20xSSC 5 ml, 50xDerhardt solution 0.4 ml, 1M sodium phosphate buffer (pH 6.5) 0.4 ml and sonication-modified salmon sperm DNA (a product of Sigmer) of 50.0 mg/ml concentration 0.1 ml to obtain a hybridization solution.

Next, the five kinds of filters having the probe nucleic acid previously prepared immobilized in spots were dipped in a mixture of 200 ml of 3xSSD and Denhardt solution (mixing volume ratio 1:1) at 42 °C for 30 minutes to effect pre-hybridization, then the previous hybridization solution (3 ml) was added therein, and the mixture was sealed within a polyethylene bag and left to stand at 42 °C.

After 12 hours, the filters were taken out, washed and the color indication reactions were carried out in conventional manner (Bioindustry, Vol. 3, No. 6, 1989, p479-504, etc.).

As the result, concerning B and C, detection was possible to the filter of No. 2, namely to 5 ng. For the filter spotted with A, no color formation was seen in all of No. 1 to No. 5.

Example 5

By use of the experimental system according to an example of the present invention of Example 2, comparison was made with a prior art example.

As an investigation according to the example of the present invention, similarly as in Example 2, a 41-mer oligonucleotide having the following DNA base sequence constituting a part of a plasmid pUC 19 (Probe A) and a 41-mer oligonucleotide having the following DNA base sequence constituting a part of a plasmid pBR 322 (Probe B) were synthesized by a DNA synthesizer (a product of ABI, Model 381 A).

Probe A

5'GATCGCCCTTCCCAACAGTTGCGCAGCCTGAATGGCGAATG3'

Probe B

5'CCGTTCAGCCCGACCGCTGCGCCTTATCCGGTAACTATCGT3'

When the purities of these synthesized products were examined according to the same operation as in Example 1, both were found to have a purity of 95 % or more. These synthesized products were used directly without purification in the subsequent operations.

A solution for spotting of each of the synthesized products at 200 ng/μl concentration was prepared, and each 2 μl of the solution was spotted onto a nitrocellulose filter (a product of Schleicher & Schanel) so that the spots of Probes A and B were arranged as shown in Fig. 1, dried and then baked at 80 °C for 2 hours.

Five sheets of such filters were prepared and named No. 1 to No. 5.

On the other hand, as a sample, a plasmid pUC 19 was labelled according to the following operations.

Labelling was carried out according to the nick translation method of Rigby et al.

Nick translation buffer 10 μl, 1 μl 2.5 mM dGTP, 1 μl 2.5 mM dATP, 1 μl 2.5 mM dTTP, biotinylated UTP 7.0 μl (BRL), 1 μl DNase (0.05 μg/ml), and a sample DNA (pUC19) 4.0 μl (0.4 μg/4μl) were placed in Eppendorf tube to make ddH$_2$O a reaction mixture of 100 μl. After the reaction at 12 °C for 10 minutes, 1 μl DNA polymeraze (5 to 10 units) was added. After the reaction was carried out at 12 °C for 75 minutes, the reaction was stopped by addition of a stopping solution. The labelled DNA was purified by phenol extraction, ethanol precipitation. The purified product was subjected to the heating treatment at 95 °C for 5 minutes, and then diluted with a reaction mixture for hybridization to amounts of 50 ng, 5 ng, 0.5 ng, 0.05 ng and 0.005 ng. The reaction mixture for hybridization used was a mixture of 100 % formamide 9 ml, 20 x SSC 5 ml, 50 x Denhardt solution 0.4 ml, 1M sodium phosphate buffer (pH 6.5) 0.4 ml, (50.0 mg/ml) sonication-modified salmon sperm (a product of Sigmer) 0.1 ml.

By use of the hybridization reaction mixtures of the five kinds of concentrations as mentioned above, hybridization was effected for the 5 sheets of filters previously prepared.

For the filter of No. 1, the reaction mixture of 50 ng was employed; for the filter of No. 2, the reaction mixture of 5 ng; for the filter of No. 3, the reaction mixture of 0.5 ng; for the filter of No. 4, the reaction mixture of 0.05 ng; and for the filter of No. 5, the reaction mixture of 0.005 ng.

First, the filter having the probe nucleic acid immobilized in spots previously prepared was dipped in 200 ml of a mixture of 3 x SSC and Denhardt solution at 42 °C for 30 minutes to effect pre-hybridization, and then the hybridization solutions of the respective concentrations were added as previously shown, and each mixture was sealed within a polyethylene bag and left to stand at 42 °C.

After 12 hours, the filters were taken out, washed and the color indication reactions were carried out in conventional manner (Bioindustry, Vol. 3, No. 6, 1989, p479-504, etc.).

As the result, for the filters of No. 1, No. 2, No. 3 and No. 4, color formation of blue violet was observed at the position spotted with Probe A. That is, color formation occurred up to 0.05 ng of No. 4. At the position spotted with Probe B, no color formation could be seen.

On the other hand, as a prior art example, a double-stranded DNA constituting a part of pUC 19 as shown below was synthesized, biotinylated dUTP was introduced at 3'-end with the T4 DNA Polymerase and the labelled product was dissociated into a single strand and used as the probe for detection according to the prior art method.

$$5'\text{ATCGCCCTTCCCAACAGTTGCGCAGCCTGAATGGCGAAT}3'$$

$$\text{TAGCGGGAAGGGTTGTCAACGCGTCGGACTTACCGCTTA}$$

Plasmid pBR 322 and plasmid pUC 19 as the sample nucleic acid were spotted respectively onto a nitrocellulose filter (a product of Schleicher & Schanel) at the amounts indicated below.

| Filter No. | | 1 | 2 | 3 | 4 | 5 |
|---|---|---|---|---|---|---|
| Sample | | | | | | |
| | | ng | ng | ng | ng | ng |
| pUC 19 | | 50 | 5 | 0.5 | 0.05 | 0.005 |
| pBR 322 | | 50 | 5 | 0.5 | 0.05 | 0.005 |

The five kinds of filters were dried and then oven-dried at 80 °C for 2 hours.

The probe previously prepared was subjected to heating treatment at 95 °C for 5 minutes to be converted into a single strand.

The reaction mixture subjected to the heating treatment was mixed with 100 % formamide 9 ml, 20xSSC 5 ml, 50xDerhardt solution 0.4 ml, 1M sodium phosphate buffer (pH 6.5) 0.4 ml and sonication-modified salmon sperm DNA (a product of Sigmer) of 50.0 mg/ml concentration 0.1 ml to obtain a hybridization solution.

Next, the five kinds of filters having the sample nucleic acid previously prepared immobilized in spots were dipped in a mixture of 200 ml of 3xSSC and Denhardt solution (mixing volume ratio 1:1) at 42 °C for 30 minutes to effect pre-hybridization, then the previous hybridization solution (3 ml) was added therein, and the mixture was sealed within a polyethylene bag and left to stand at 42 °C.

After 12 hours, the filters were taken out, washed and the color indication reactions were carried out in conventional manner (Bioindustry, Vol. 3, No. 6, 1989, p479-504, etc.).

As the result, concerning the filters of No. 1 and No. 2, color formation of blue violet was seen at the position spotted with the sample of pUC 19. That is, color formation occurred up to 5 ng of No. 2.

No color formation could be seen at all the positions spotted with pBR 322.

Example 6

By use of the experimental system according to an example of the present invention of Example 3, comparison was made with a prior art example.

The probes employed are the same Probes 1 to 4 as in Example 3, and as the sample, Sample A suspected of no phenylalaninuria judged in Example 3, and Sample B suspected of phenylalaninuria controlled to predetermined concentrations were used.

As an investigation according to the example of the present invention, a nitrocellulose filter spotted with the respective probes at the arrangements shown in Fig. 2 similarly as in Example 3 was prepared.

Hybridization solutions having the amounts of 500 ng, 50 ng, 5 ng, 0.5 ng, 0.05 ng and 0.005 ng of the DNA applied with the same pre-treatment and label as in Example 3 as a sample were prepared.

By use of the hybridization solution together with the probe-immobilized filter previously prepared, pre-hybridization and hybridization by use of the hybridization solutions with the respective concentrations were conducted according to the same operations as in the item 1-3) in Example 1 except that the hybridization conditions were made 50 °C, 16 hours.

After completion of the reaction, the filter was subjected to washing twice with 2 x SSPE containing 0.1 % SDS at room temperature for 30 minutes, further once with 5 x SSPE containing 0.1 % SDS at 55 °C for 10 minutes.

When the filters after washing were subjected to the color formation reaction of biotinylated nucleic acid in conventional manner, the following results were obtained.

Sample A

|  | 500ng | 50ng | 5ng | 0.5ng | 0.05ng | 0.005ng |
|---|---|---|---|---|---|---|
| Probe 1 | o | o | o | o | o | x |
| " 2 | x | x | x | x | x | x |
| " 3 | o | o | o | o | o | x |
| " 4 | x | x | x | x | x | x |

Sample B

|  | 500ng | 50ng | 5ng | 0.5ng | 0.05ng | 0.005ng |
|---|---|---|---|---|---|---|
| Probe 1 | x | x | x | x | x | x |
| " 2 | x | x | x | x | x | x |
| " 3 | x | x | x | x | x | x |
| " 4 | o | o | o | o | x | x |

Note:　o ... color formation

　　　x ... no color formation

On the other hand, as the prior art example, first, the probes 1 to 4 were labelled. The labelling method was performed by synthesizing a complementary double-stranded DNA corresponding to the probes 1 to 4, introducing biotinylated dUTP at 3'-end with T4 DNA polymerase, and dissociating the labelled product into a single strand, which was used as the probe.

The sample nucleic acids A and B were spotted onto a nitrocellulose filter at the concentrations shown below in Table 1.

| Filter No. Sample | No.1 | No.2 | No.3 | No.4 | No.5 | No.6 |
|---|---|---|---|---|---|---|
| | ng | ng | ng | ng | ng | ng |
| A | 500 | 50 | 5 | 0.5 | 0.05 | 0.005 |
| B | 500 | 50 | 5 | 0.5 | 0.05 | 0.005 |

The six kinds of filters were dried and then over-dried at 80 °C for 2 hours.

The Probes 1 to 4 previously prepared were subjected to heating treatment at 95 °C for 5 minutes to be converted into single strands.

The reaction mixture subjected to the heating treatment was mixed with 100 % formamide 9 ml, 20xSSC 5 ml, 50xDerhardt solution 0.4 ml, 1M sodium phosphate buffer (pH 6.5) 0.4 ml and sonication-modified salmon sperm DNA (a product of Sigmer) of 50.0 mg/ml concentration 0.1 ml to obtain four kinds of hybridization solutions.

Next, the six kinds of filters having the probe nucleic acid previously prepared immobilized in spots were dipped in a mixture of 200 ml of 3xSSC and Denhardt solution (mixing volume ratio 1:1) at 42 °C for 30 minutes to effect pre-hybridization, then each of the previous hybridization solutions (3 ml) was added therein, and the mixture was sealed within a polyethylene bag and left to stand at 42 °C.

After 12 hours, the filters were taken out, washed and the color indication reactions were carried out in conventional manner (Bioindustry, Vol. 3, No. 6, 1989, p479-504, etc.).

The results are shown below in Tables.

Probe 1

| | 500ng | 50ng | 5ng | 0.5ng | 0.05ng | 0.005ng |
|---|---|---|---|---|---|---|
| Sample A | o | x | x | x | x | x |
| Sample B | x | x | x | x | x | x |

Probe 2

| | 500ng | 50ng | 5ng | 0.5ng | 0.05ng | 0.005ng |
|---|---|---|---|---|---|---|
| Sample A | x | x | x | x | x | x |
| Sample B | x | x | x | x | x | x |

Probe 3

| | 500ng | 50ng | 5ng | 0.5ng | 0.05ng | 0.005ng |
|---|---|---|---|---|---|---|
| Sample A | o | o | x | x | x | x |
| Sample B | x | x | x | x | x | x |

Probe 4

| | 500ng | 50ng | 5ng | 0.5ng | 0.05ng | 0.005ng |
|---|---|---|---|---|---|---|
| Sample A | x | x | x | x | x | x |
| Sample B | o | x | x | x | x | x |

Note:   o ... color formation

x ... no color formation

In the present invention, no requirement for labelling of the probe nucleic acid is demanded, since the sample nucleic acid is labelled and the probe nucleic acid is not labelled. As the result, a short nucleotide chain length which is readily available and also can be prepared to high precision can be utilized, and detection can be done by simple operation even at low concentration. Also, since sample nucleic acid is generally one having long nucleotide chain length, by employment of labelling onto sample nucleic acid, it becomes possible to effect efficient labelling by use of a nonradioactive label, whereby labelling and detection operation become safer and simpler.

Further, when detections of plural number of nucleic acids are performed at the same time by use of plural kinds of probe nucleic acids, in the method of labelling probe nucleic acids of the prior art, cumbersome operations of performing differentiation of plural number of probe nucleic acids by use of plural different kinds of labels have been required, but according to the present invention, labelling of the sample nucleic acid can be effected with one kind of label, whereby labelling operations can be simplified and detections of plural kinds of nucleic acids can be done simply and efficiently.

The present invention provides a nucleic acid detection method to detect the presence of a nucleic acid to be detected in a sample nucleic acid, upon detecting a formation of a nucleic acid to be detected-probe nucleic acid hybrid in the reaction between the sample nucleic acid and a probe nucleic acid, wherein said sample nucleic acid is applied with a label available for detecting said hybrid.

## Claims

1. A method to detect the presence of a nucleic acid to be detected in a sample nucleic acid comprising the steps of
   (a) providing a probe nucleic acid which is an oligonucleotide with a nucleotide chain length of 25 bases or less capable of detecting a point mutation of a genetic disease, immobilized on a carrier;
   (b) labelling the said sample nucleic acid;
   (c) adding the labelled sample nucleic acid to the immobilized probe nucleic acid;
   (d) detecting the point mutation by detecting the presence of the said nucleic to be detected in the said sample nucleic acid bound to the said probe nucleic acid.

2. The method according to claim 1, wherein the nucleic acid to be detected has sequences inherent in a disease selected from the group consisting of a genetic disease, cancer and viral infections.

3. The method according to claim 1, wherein step (a) further comprises immobilization of multiple types of probes at predetermined locations on the carrier.

## Patentansprüche

1. Verfahren zum Nachweis des Vorhandenseins einer Nucleinsäure, nachzuweisen in einer Nucleinsäureprobe, folgende Schritte umfassend:
   (a) Bereitstellung einer Nucleinsäure-Sonde, die ein Oligonucleotid mit einer Nucleotid-Kettenlänge aus 25 Basen oder weniger darstellt, geeignet zum Nachweis einer Punktmutation einer genetischen Krankheit, immobilisiert auf einem Träger;
   (b) Markieren der Nucleinsäureprobe;
   (c) Zugabe der markierten Nucleinsäureprobe zu der immobilisierten Nucleinsäure-Sonde;
   (d) Nachweis der Punktmutation mittels Nachweises des Vorhandenseins der in der an die Nucleinsäure-Sonde gebundenen Nucleinsäureprobe nachzuweisenden Nucleinsäure.

2. Verfahren nach Anspruch 1, wobei die nachzuweisende Nucleinsäure einer Krankheit inhärente Sequenzen aufweist, ausgewählt aus der Gruppe bestehend aus genetischer Krankheit, Krebs und Virusinfektionen.

3. Verfahren nach Anspruch 1, wobei Schritt (a) ferner die Immobilisierung multipler Typen von Sonden an festgelegten Stellen auf dem Träger umfaßt.

## Revendications

1. Procédé pour détecter la présence d'un acide nucléique à détecter dans un échantillon d'acide nucléique, comprenant les étapes
   (a) d'utilisation d'un acide nucléique servant de sonde, qui est un oligonucléotide ayant une longueur de chaîne de nucléotides égale ou inférieure à 25 bases capable de détecter une mutation ponctuelle d'une maladie génétique, immobilisé sur un support ;
   (b) de marquage dudit acide nucléique de l'échantillon ;
   (c) d'addition de l'acide nucléique marqué de l'échantillon à l'acide nucléique immobilisé servant de sonde ;
   (d) de détection de la mutation ponctuelle par détection de la présence dudit acide nucléique à détecter dans ledit échantillon d'acide nucléique lié audit acide nucléique servant de sonde.

2. Procédé suivant la revendication 1, dans lequel l'acide nucléique à détecter possède des séquences propres à une maladie choisie dans le groupe consistant en une maladie génétique, un cancer et des infections virales.

3. Procédé suivant la revendication 1, dans lequel l'étape (a) comprend en outre l'immobilisation de types multiples de sondes à des emplacements prédéterminés sur le support.

●: PROBE A
x: PROBE B

# F I G. I

PROBE 1    PROBE 2    PROBE 3    PROBE 4

# F I G. 2